# EUROPEAN PATENT SPECIFICATION

(11) **EP 1 876 181 B1**
(45) Date of publication and mention of the grant of the patent: **24.07.2013**
(21) Application number: 06745829.9
(22) Date of filing: 28.04.2006
(51) Int. Cl.: C07F 9/24, C07F 9/54, H01L 31/04, H01M 8/02, H01M 14/00

(54) **IONIC LIQUID CONTAINING PHOSPHONIUM ION AND METHOD FOR PRODUCING SAME**
IONISCHE FLÜSSIGKEIT ENTHALTENDES PHOSPHONIUMION UND HERSTELLUNGSVERFAHREN DAFÜR
LIQUIDE IONIQUE CONTENANT L ION PHOSPHONIUM ET MÉTHODE DE PRODUCTION DUDIT LIQUIDE

(30) Priority: 28.04.2005 WO PCT/JP2005/008229
(43) Date of publication of application: 09.01.2008
(73) Proprietor: Kanto Denka Kogyo Co., Ltd., Chiyoda-ku Tokyo 101-0063 (JP)
(72) Inventor: SUETO, Kumiko, Kanai, Shibukawa-shi, Gunma 3770027 (JP); OMAE, Osamu, Kanai, Shibukawa-shi, Gunma 3770027 (JP); GAO, Yuan, Kanai, Shibukawa-shi,Gunma 3770027 (JP)
(74) Representative: Forstmeyer, Dietmar
(86) International application number: PCT/JP2006/308948
(87) International publication number: WO 2006/118232

(56) References cited:
- WO-A1-02/076924
- US-A- 2 774 658
- US-A- 5 541 287
- JOHN R. VAN WAZER, CLAYTON F. CALLIS, JAMES N. SHOOLERY, ROBERT C. JONES: "Principles of Phosphorus Chemistry. II. Nuclear Magnetic Resonance Measurements" J. AM. CHEM. SOC, vol. 78, no. 22, 1956, pages 5715-5726, XP002603450
- CASTRO B. ET AL.: "Sels d'atdp - 20:Relations reactivite-structure dans les cinetiques de decomposition de sels d'atdp substitues. Hypersensibilite a des substituants en position 3 de la substitution nucleophile SN2" TETRAHEDRON, vol. 35, no. 4, 1979, pages 627-632, XP002603451
- SPENCER ROACH L ET AL: "Controlling Nonspecific Protein Adsorption in a Plug-Based Microfluidic System by Controlling Interfacial Chemistry Using Fluorous-Phase Surfactants" ANALYTICAL CHEMISTRY, AMERICAN CHEMICAL SOCIETY, US LNKD- DOI:10.1021/AC049061W, vol. 77, no. 3, 1 February 2005 (2005-02-01), pages 785-796, XP002527289 ISSN: 0003-2700 [retrieved on 2004-12-30]
- PETZ W. ET AL.: 'The reactions of CH2:P(NMe2)3 with Fe(CO)5, Cr(CO)6, and CS2. Molecular structures of [MeP(NMe2)3][(CO)5CrC(0)CH:P(NMe2)3] and (CO)4Fe:C(OMe)CH:P(NMe2)3' ZEITSCHRIFT FUER ANORGANISCHE UND ALLGEMEINE CHEMIE vol. 624, no. 7, 1998, pages 1123 - 1129, XP003003061
- KIM T.C. ET AL.: 'Synthesis of new bis(dialkylamino)-1,4,2(lambda 4)-diazaphospholanium salts from phosphenium cations and imines' TETRAHEDRON LETTERS vol. 31, no. 31, 1990, pages 4459 - 4462, XP003003062
- HAYNES R.K. ET AL.: 'The preparation of (methylthio)- and (methylseleno)tri(alkyl or aryl)phosphonium salts and their reactions with carboxylic acid and alcohols' AUSTRALIAN JOURNAL OF CHEMISTRY vol. 37, no. 6, 1984, pages 1183 - 1194, XP003003063

## Description

### Technical Field

The present invention relates to an ionic liquid that is in a liquid state in a wide range of temperatures from low temperatures, having a low viscosity and an excellent electrochemical stability, a method for producing the same, and an application thereof including electric storage devices, rechargeable lithium batteries, electrical double layer capacitors, dye sensitized solar cells, fuel cells and reaction solvents.

### Background Art

Many ionic liquids that contain a nitrogen atom-containing onium cation such as typically an ammonium cation have been reported so far. They are in a liquid state at a temperature over 25°C, but at 25°C or lower only a few ionic liquids can keep the liquid state. In addition, the ionic liquid that has a high viscosity around room temperature and is difficult to be used as an electrolyte or solvent by itself, has been only reported so far (see Patent Documents 1 and 2, and Non-Patent Documents 1 to 3).
Further, among ionic liquids that contain a cation having relatively low viscosity and melting point such as an imidazolium cation, many ionic liquids have a difficulty of being used as an electrolyte for electric storage devices because of inadequate stabilities caused by their low stability against reduction and narrow potential window (see Patent Document 3 and Non-Patent Documents 4 and 5).

A large stumbling block for the application of the ionic liquids to rechargeable lithium batteries; electrical double layer capacitors; fuel cells; dye sensitized solar cells; or the electrolytes, electrolyte solutions or additives for electric storage devices is that there are very few ionic liquids keeping a stable liquid state in a wide range of temperatures from low temperatures, having low viscosity and high conductivity, being excellent in electrochemical stability, and usable by itself.

Patent Document 1: International Publication No. WO 02/076924 Pamphlet
Patent Document 2: Japanese Patent Laid-Open Publication No. 2003-331918
Patent Document 3: Japanese Patent Application Laid-Open No. 2001-517205
Non-Patent Document 1: Hajime Matsumoto and Yoshinori Miyazaki, YOYUEN OYOBI KOONKAGAKU, Vol. 44, p. 7 (2001)
Non-Patent Document 2: H. Matsumoto, M. Yanagida, K. Tanimoto, M. Nomura, Y. Kitagawa and Y. Miyazaki, Chem. Lett, Vol. 8, p. 922 (2000)
Non-Patent Document 3: D. R. MacFarlane, J. Sun, J. Golding, P. Meakin and M. Forsyth, Electrochemica Acta, Vol. 45, p. 1271 (2000)
Non-Patent Document 4: Rika Hagiwara, Electrochemistry, Vol. 70, No. 2, p. 130 (2002)
Non-Patent Document 5: Y. Katayama, S. Dan, T. Miura and T. Kishi, Journal of The Electrochemical Society, Vol. 148 (2), C102-C105 (2001)

Bertrand Castro, et al., in Tetrahedron 1979, Vol. 35, No. 4, pages 627 to 632, describe the kinetics of decomposition of various salts comprising Alkyloxy-Tris-Dimethylamino-Phosphonium (ATDP) as cation component and chloride as anion component in different solvents.

### Disclosure of the Invention

### Problems to be Solved by the Invention

An object of the present invention is to provide an ionic liquid having low viscosity, adequate conductivity and excellent electrochemical stability, and a method for producing the ionic liquid. Further, an object of the present invention is to provide an ionic liquid as described above that can be used for electrolyte solutions, rechargeable lithium batteries, electrical double layer capacitors, dye sensitized solar cells, fuel cells, reaction solvents and the like, particularly to provide an ionic liquid that is stably in a liquid state at around room temperature, specifically to provide an ionic liquid containing novel phosphonium cations.

### Means for Solving the Problems

The present inventors have synthesized a number of salts composed of cation components and anion components and have made intensive studies on an ionic liquid to achieve the above objectives. As a result, the present inventors have found that an ionic liquid containing as a cation component one or plural kinds of components selected from the group consisting of organic cations represented by the following general formula (1) has low viscosity, adequate conductivity, and excellent electrochemical stability.

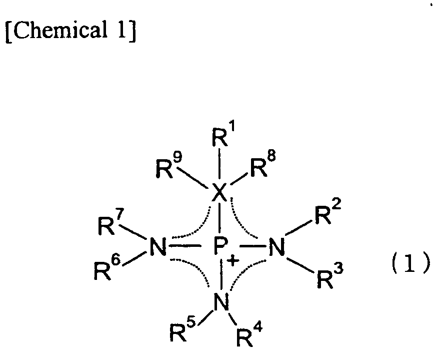

wherein substitution groups R¹ to R⁹ may be independently the same or different from one another; each of the substitution groups R¹ to R⁹ is a hydrogen atom, a straight chain or branched chain alkyl group having 1 to 30 carbon atoms, a straight chain or branched chain alkenyl group having 2 to 30 carbon atoms with one or plural double bonds, a straight chain or branched chain alkynyl group having 2 to 30 carbon atoms with one or plural triple bonds, a saturated or a partially or fully unsaturated cycloalkyl group, an aryl group, or a heterocyclic group; any hydrogen atoms contained in one or a plurality of the substitution groups R¹ to R⁹ may be partially or fully substituted by a halogen atom, or partially substituted by a CN group or a NO₂ group; any one of the substitution groups R¹ to R⁹ may form a ring structure together with one another; any carbon atoms contained in the substitution groups R¹ to R⁹ may be substituted by an atom and/or an atomic group selected from the group consisting of -O-, -C(O)-, -C(O)O-, -S-, -S(O)-, -SO₂-, -SO₃-, -N=, -N=N-, -NH-, -NR'-, -N(R')₂, -PR'-, -P(O)R'-, -P(O)R'-O-, -O-P(O)R'-O-and -P(R')₂=N-, wherein R' is a straight chain or branched chain alkyl group having 1 to 10 carbon atoms, an alkyl group partially or fully substituted by a fluorine atom, a saturated or a partially or fully unsaturated cycloalkyl group, a non-substituted or substituted phenyl group, or a non-substituted or substituted heterocyclic group; X represents a sulfur atom, an oxygen atom or a carbon atom; R⁸ and R⁹ exist only when X is a carbon atom; when X is a carbon atom, X, R¹, R⁸ and R⁹ may form a saturated or a partially or fully unsaturated ring structure together with one another; and a dashed line represents a conjugated structure.

Namely, the above objectives of the present invention have been accomplished by providing "an ionic liquid comprising a cation component and an anion component, and the cation component is one or plural kinds selected from the group consisting of cation components represented by the general formula (1)", and the anion component is as defined in the claims.

In particular, the present invention provides the following:
[1] An ionic liquid comprising a cation component and an anion component, the cation component being one or plural kinds selected from the group consisting of cation components represented by the following formula (1): 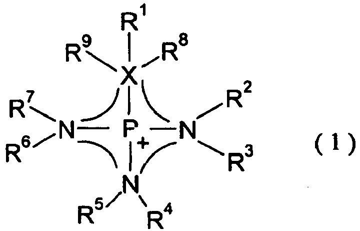 wherein substitution groups R¹ to R⁹ may be independently the same or different from one another; X represents a carbon atom or an oxygen atom; R⁸ and R⁹ exist only when X is a carbon atom; and a dashed line represents a conjugated structure,
   wherein, if X is a carbon atom, each of R² to R⁷ is a straight chain alkyl group having 1 to 4 carbon atoms; each of R⁸ and R⁹ is a hydrogen atom; and R¹ is a straight chain or branched chain alkyl group having 1 to 10 carbon atoms or a straight chain or branched chain alkoxy group having 1 to 10 carbon atoms; and the anion component is (CF₃SO₂)₂N⁻, PF₆ or BF₄⁻; or
   wherein, if X is an oxygen atom, each of R¹ to R⁷ is a straight chain or branched chain alkyl group having 1 to 10 carbon atoms, and may be the same or different from one another; and the anion component is one or plural kinds selected from the group consisting of [R^{f}SO₃]⁻, [(R^{f}SO₂)₂N]⁻, CF₃SO₃⁻, CF₃COO⁻, PF₆⁻, BF₄⁻, [N(CN)₂]⁻, [AlCl₄]⁻, SO₄²⁻, HSO₄⁻, NO₃⁻, F⁻, Br and I⁻; wherein R^{f} is a fluorine-containing substitution group.
[2] The ionic liquid according to the above [1], wherein in the general formula (1), X is an oxygen atom, each of R² to R⁷ is a straight chain alkyl group having 1 to 4 carbon atoms; R¹ is a straight chain or branched chain alkyl group having 1 to 10 carbon atoms ; and the anion component is (CF₃SO₂)₂N⁻, PF₆ or BF₄⁻.
[3] The ionic liquid according to the above [1], wherein in the general formula (1), X is a carbon atom, each of R² to R⁷ is a straight chain alkyl group having 1 to 4 carbon atoms; each of R⁸ and R⁹ is a hydrogen atom; and R¹ is a straight chain or branched chain alkyl group having 1 to 10 carbon atoms or a straight chain or branched chain alkoxy group having 1 to 10 carbon atoms; and the anion component is (CF₃SO₂)₂N⁻ or PF₆⁻.
[4] An electric storage device comprising the ionic liquid according to any of the above [1] to [3] as an electrolyte solution.
[5] A rechargeable lithium battery comprising the ionic liquid according to any of the above [1] to [3].
[6] An electrical double layer capacitor comprising the ionic liquid according to any of the above [1] to [3].
[7] A dye sensitized solar cell comprising the ionic liquid according to any of the above [1] to [3].
[8] A fuel cell comprising the ionic liquid according any of the above [1] to [3].
[9] A reaction solvent comprising the ionic liquid according to any of the above [1] to [3].
[10] A method for producing an ionic liquid comprising alkylating an organic substance represented by the following general formula (2),
the ionic liquid containing an organic substance represented by the general formula (1) as a cation component, 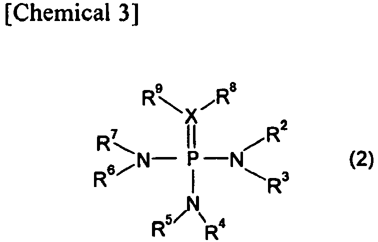 wherein substitution groups R² to R⁹ may be independently the same or different from one another; X represents a carbon atom or an oxygen atom; and R⁸ and R⁹ exist only when X is a carbon atom,
wherein, if X is a carbon atom, each of R² to R⁷ is a straight chain alkyl group having 1 to 4 carbon atoms; each of R⁸ and R⁹ is a hydrogen atom; and R¹ is a straight chain or branched chain alkyl group having 1 to 10 carbon atoms or a straight chain or branched chain alkoxy group having 1 to 10 carbon atoms; or
wherein, if X is an oxygen atom, each of R² to R⁷ is a straight chain or branched chain alkyl group having 1 to 10 carbon atoms, and may be the same or different from one another.

[11] A method for producing an ionic liquid according to the above [10] comprising anion exchange with a resulting salt after the alkylation.

### Brief Description of the Drawings

[FIG. 1] Figure 1 is a graph showing a CV curve of tri(dimethylamino) butoxyphosphonium bistrifluromethane sulfonylimide in Example 3.
[FIG. 2] Figure 2 is a graph showing a CV curve of tri(dimethylamino) butylphosphonium bistrifluoromethane sulfonylimide in Example 4.

### Best Mode for Carrying Out the Invention

As the cation component represented by the general formula (1), the substitution groups R¹ to R⁹ in the general formula (1), are as defined in the claims. Further, X in the general formula (1) is an oxygen atom, or a carbon atom.

The anion component is one or plural kinds selected from the group consisting of [RSO₃⁻], [R^{f}SO₃]⁻, [(R^{f}SO₂)₂N]⁻, [(R^{f}SO₂)₃C]⁻, [(FSO₂)₃C]⁻, [RCH₂OSO₃]⁻, [RC(O)O]⁻, [R^{f}C(O)O]⁻, [CCl₃C(O)O]⁻, [(CN)₃C]⁻, [(CN)₂CR]⁻, [(RO(O)C)₂CR]⁻, [R₂P(O)O]⁻, [RP(O)O₂]²⁻, [(RO)₂P(O)O]⁻, [(RO)P(O)O₂]²⁻, [(RO)(R)P(O)O]⁻, [R^{f}₂P(O)O]⁻, [R^{f}P(O)O₂]²⁻, [B(OR)₄]⁻, [N(CF₃)₂]⁻, [N(CN)₂]⁻, [AlCl₄]⁻, PF₆⁻, BF₄⁻, SO₄²⁻, HSO₄⁻, NO₃⁻, F⁻, Cl⁻, Br and I⁻, wherein each of the substitution groups Rs is a hydrogen atom, a halogen atom, a C₁₋₁₀ straight chain or branched chain alkyl group, a C₂₋₁₀ straight chain or branched chain alkenyl group having one or plural double bonds, a C₂₋₁₀ straight chain or branched chain alkynyl group having one or plural triple bonds, or a saturated or a partially or fully unsaturated cycloalkyl group; any hydrogen atoms contained in these substitution groups Rs may be partially or fully substituted by a halogen atom, or partially substituted by a CN group or a NO₂ group; any carbon atom that is contained in these Rs may be substituted by an atom and/or an atomic group selected from the group consisting of -O-, -C(O)-, -C(O)O-, -S-, -S(O)-, -SO₂-, -SO₃-, -N=, -N=N-, -NR'-, -N(R')₂, -PR'-, -P(O)R'-, -P(O)R'-O-, -O-P(O)R'-O-, and -P(R')₂=N-, wherein R' is a C₁₋₁₀ straight chain or branched chain alkyl group, an alkyl group partially or fully substituted by a fluorine atom, a saturated or a partially or fully unsaturated cycloalkyl group, a non-substituted or substituted phenyl group, or a non-substituted or substituted heterocyclic group; and R^{f} is a fluorine-containing substitution group. These anion components are combined with the aforementioned cation components and provide an ionic liquid having low viscosity, adequate conductivity, and excellent electrochemical stability.

The anion component used in the present invention as a counter ion of the cation component represented by the general formula (1) is one or plural kinds selected from the group consisting of [R^{f}SO₃]⁻, [(R^{f}SO₂)₂N]⁻, CF₃SO₃⁻, CF₃COO⁻, PF₆⁻, BF₄⁻, [N(CN)₂]⁻, [AlCl₄]⁻, SO₄²⁻, HSO₄⁻, NO₃⁻, F⁻, Br⁻ and I⁻, and more preferably one or plural kinds selected from the group consisting of [R^{f}SO₃]⁻, [(R^{f}SO₂)₂N]⁻, CF₃SO₃⁻, CF₃COO⁻, [N(CN)₂]⁻, [AlCl₄]⁻, SO₄²⁻ , HSO₄⁻ and NO₃⁻.
The combination of the aforementioned cation components and these anion components is capable of providing an ionic liquid having still more preferable properties, namely a stable liquid state in a wide range of temperatures from low temperatures, low viscosity, adequate conductivity, and excellent electrochemical stability.

In the more preferable case, as X in the cation component represented by the general formula (1) is an oxygen atom. The ionic liquid substituted by these atoms has a low melting point. Still more preferable is the ionic liquid having an oxygen atom as X.

When an ionic liquid is prepared while placing importance on low viscosity, a specific cation component is required to be selected in such a manner that when X is an oxygen atom, each of R² to R⁷ is a straight chain alkyl group having 1 to 4 carbon atoms; R¹ is a straight chain or branched chain alkyl group having 1 to 10 carbon atoms ; and the anion component is (CF₃SO₂)₂N⁻, PF₆⁻ or BF₄⁻; or when X is a carbon atom, each of R² to R⁷ is a straight chain alkyl group having 1 to 4 carbon atoms; each of R⁸ and R⁹ is a hydrogen atom; and R¹ is a straight chain or branched chain alkyl group having 1 to 10 carbon atoms or a straight chain or branched chain alkoxy group having 1 to 10 carbon atoms; and the anion component is (CF₃SO₂)₂N⁻ or PF₆⁻. With these combinations, ionic liquid that exhibits a stable liquid state in a wide range of temperatures from low temperatures, having low viscosity, adequate conductivity, and excellent electrochemical stability can be obtained.

The ionic liquid of the present invention exhibits excellent conductivity, having low viscosity and excellent electrochemical stability as well. Due to these excellent performances, the ionic liquid of the present invention is used as a material for the electrolytes, electrolyte solutions, additives and others for electric storage devices; rechargeable lithium batteries; electrical double layer capacitors; fuel cells; and dye sensitized solar cells, and is also used as a reaction solvent for various reactions. Note that, such an ionic liquid that has both low viscosity and electrochemical stability has not been attainable so far. The ionic liquid of the present invention precisely satisfies both of these properties.
Here, the cation component represented by the general formula (1) shows a phosphonium cation having a plus charge on the phosphorus atom for convenience in writing, but the plus charge may be delocalized in the molecule depending on the kind of the hetero atom represented by X.

A typical method for synthesizing an ionic liquid that contains the cation component represented by the general formula (1) will be mentioned below.

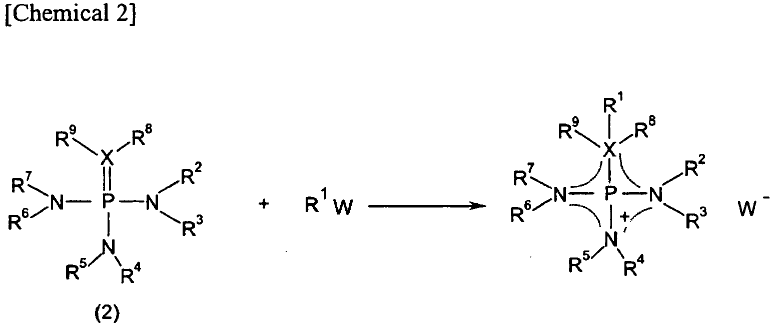

To a source organic substance represented by the general formula (2), an alkylation agent (R'W) is added dropwise and reacted at a predetermined temperature for a predetermined time. After the reaction mixture is washed with diethylether or the like, it is dried under vacuum. The alkylation agent (R¹W) may include dialkylsulfate, dialkylsulfonate, dialkylcarbonate, trialkylphosphate, alkylmono fluoroalkylsulfonate, alkylpolyfluoroalkylsulfonate,fluoroalkylsulfonate, alkylperfluoroalkylsulfonate, alkylmonofluorocarboxylate, alkylpolyfluorocarboxylate, alkylperfluorocarboxylate, alkyliodide, alkylbromide, alkylchloride, sulfuric acid, nitric acid and hydrochloric acid.

Further, for example, an ionic liquid having a different kind of anion can be obtained by anion exchange as shown below.

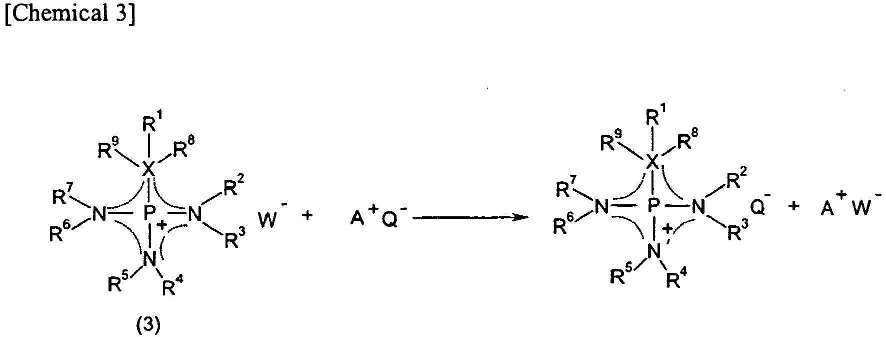

Here, the ionic bonding compound AQ may include, for example, LiN(CF₃SO₂)₂, NaN(CF₃SO₂)₂, KN(CF₃SO₂)₂, CF₃SO₃Li, CF₃SO₃Na, CF₃SO₃K, CF₃CH₂SO₃Li, CF₃CH₂SO₃Na, CF₃CH₂SO₃K, CF₃COOLi, CF₃COONa, CF₃COOK, LiPF₆, NaPF₆, KPF₆, LiBF₄, NaBF₄, KBF₄, LiSbF₆, NaSbF₆, KSbF₆, NaN(CN)₂, AgN(CN)₂, Na₂SO₄, K₂SO₄, NaNO₃ and KNO₃, but it is not limited to these compounds.

In the general formula (3), the substitution groups R¹ to R⁹ may be independently the same or different from one another. The substitution groups R¹ to R⁹ each are a hydrogen atom, a halogen atom, a C₁₋₃₀ straight chain or branched chain alkyl group, a C₂₋₃₀ straight chain or branched chain alkenyl group having one or plural double bonds, a C₂₋₃₀ straight chain or branched chain alkynyl group having one or plural triple bonds, a saturated or a partially or fully unsaturated cycloalkyl group, an aryl group, or a heterocyclic group. Any hydrogen atoms contained in one or a plurality of these substitution groups R¹ to R⁹ may be partially or fully substituted by a halogen atom or may be partially substituted by a CN group or a NO₂ group. Any substitution groups of R¹ to R⁹ may form a ring structure together with one another. Any carbon atoms contained in the substitution groups R¹ to R⁹ may be substituted by an atom and/or an atomic group selected from the group consisting of -O-, -C(O)-, -C(O)O-, -S-, -S(O)-, -SO₂-, -SO₃-, -N=, -N=N-, -NH-, -NR'-, -N(R')₂, -PR'-, -P(O)R'-, -P(O)R'-O-, -O-P(O)R'-O- and -P(R')₂=N-, wherein R' is a C₁₋₁₀ straight chain or branched chain alkyl group, an alkyl group partially or fully substituted by a fluorine atom, a saturated or a partially or fully unsaturated cycloalkyl group, a non-substituted or substituted phenyl group, or a non-substituted or substituted heterocyclic group. X represents a sulfur atom, an oxygen atom or a carbon atom. R⁸ and R⁹ exist only when X is a carbon atom. When X is a carbon atom, X, R¹, R⁸ and R⁹ may form a saturated or a partially or fully unsaturated ring structure together with one another.

The halogen atom described above may include F, Cl, Br and I.
The cycloalkyl group described above may include cyclopropyl, cyclobutyl, cyclopentyl, cyclohexyl, cycloheptyl, cyclooctyl, cyclononyl and cyclodecyl. The cycloalkyl group may include a group that has an unsaturated bond such as a cycloalkenyl group and a cycloalkynyl group. A hydrogen atom of the cycloalkyl group may be partially or fully substituted by a halogen atom, or partially substituted by a CN group or a NO₂ group.

The heterocyclic group described above may include a group of pyrodinyl, pyrolinyl, imidazolidinyl, imidazolinyl, pyrazolidinyl, pyrazonyl, piperydyl, piperadinyl, morpholinyl or thienyl. Further, these heterocyclic groups may have one or a plurality of an alkyl group, an alkoxy group, a hydroxyl group, a carboxyl group, an amino group, an alkylamino group, a dialkylamino group, a thiol group and an alkylthio group, and a halogen atom.

The aryl group described above may include a group of phenyl, cumenyl, mesityl tolyl, xylyl or the like. These aryl groups may have one or a plurality of an alkyl group, an alkoxy group, a hydroxyl group, a carboxyl group, an acyl group, a formyl group, an amino group, an alkylamino group, a dialkylamino group, a thiol group, an alkylthio group, and a halogen atom.

Further, the substitution groups R₁ to R₉ may include an alkoxyalkyl group such as methoxymethyl, methoxyethyl, ethoxymethyl and ethoxyethyl, and the like.
Still further, as the heteroatom represented by X in the formula, there may be mentioned a sulfur atom, an oxygen atom or a carbon atom. Particularly preferably, there may be mentioned a sulfur atom or an oxygen atom. By substituting the atom, an ionic liquid having a still lower melting point can be obtained. As the anion component Q that is reacted with the compound represented by the general formula (3) and is used in combination, there may be listed the aforementioned anion components.

### Example

The present invention will be further described in detail with reference to the following examples, but it should be construed that the present invention is in no way limited to those examples.

### Example 1

### (a) Preparation of tri(dimethylamino) methoxyphosphonium methylsulfate

In an eggplant-shaped two-neck flask equipped with a reflux condenser, a dropping funnel and a magnetic stirrer, 1.4 g (11.2 mmol) of dimethylsulfate were added dropwise to 2.0 g (11.2 mmol) of hexamethylphosphate triamide at room temperature in a nitrogen gas atmosphere. After 15-hour stirring at room temperature, a white solid salt was obtained. The salt was washed sufficiently with ether and vacuum-dried at 50°C for 5 hours to obtain tri(dimethylamino) methoxyphosphonium methylsulfate with 74% yield.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: acetone-d6, reference material: tetramethylsilane)
δ 4.06 (d, 3H)
3.47 (s, 3H)
2.90 (d, 18H)
The structural formula is shown below (a dashed line in the formula represents a conjugated structure).

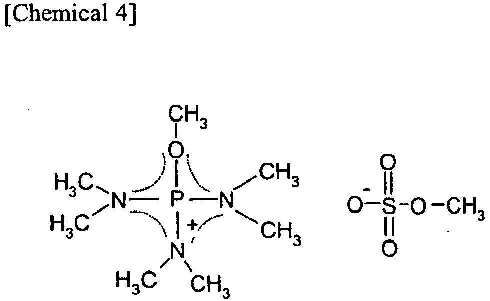

### (b) Preparation of tri(dimethylamino) methoxyphosphonium bistrifluoromethane sulfonylimide

In 100 ml of pure water, 3.05 g (10.0 mmol) of tri(dimethylamino) methoxyphosphonium methylsulfate obtained in (a) were dissolved. After impurities were extracted with CH₂Cl₂, an aqueous solution dissolving 2.87 g (10.0 mmol) of lithium bistrifluoromethane sulfonylimide in 100 ml of pure water was added to the resulting aqueous solution while stirring. After 60-minute continuous stirring, the resulting hydrophobic white solid was washed with water two or three times, extracted with dichloromethane, and purified with an alumina column. The extract was concentrated, and then vacuum-dried at 80°C for 10 hours to obtain 4.50 g (yield: 95%) of a product compound that was a white solid at room temperature and a colorless transparent liquid at 130°C.
The compound was identified with a nuclear magnetic resonance spectrometer (BRUKER Ultra Shield 300 NMR Spectrometer, supplied by BRUKER Corp.). The compound was identified as the objective compound of tri(dimethylamino) methoxyphosphonium bistrifluoromethane sulfonylimide. The spectrum data are shown below.
¹H-NMR (300 MHz, solvent: acetone-d6, reference material: tetramethylsilane) δ 4.06 (d, 3H)
2.90 (d, 18H)
¹⁹F-NMR (282 MHz, solvent: acetone-d6, reference material: CF₃Cl)
δ -79.93 (s, 6F)
The structural formula is shown below (a dashed line in the formula represents a conjugated structure).

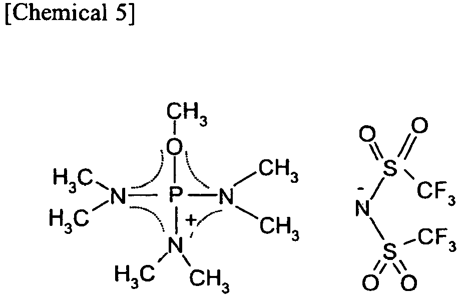

The melting point was measured with a scanning differential calorimeter (DSC8230, supplied by Shimadzu Corp.). The melting point was 127°C.

### Example 2

### (c) Preparation of tri(dimethylamino) ethoxyphosphonium ethylsulfate

In an eggplant-shaped two-neck flask equipped with a reflux condenser, a dropping funnel and a magnetic stirrer, 2.1 g (13.4 mmol) of diethylsulfate were added dropwise to 2.0 g (11.2 mmol) of hexamethylphosphate triamide at room temperature in a nitrogen gas atmosphere. After 5 day-stirring at 20°C, a white solid salt was obtained. The salt was washed sufficiently with ether and vacuum-dried at 50°C for 5 hours to obtain tri(dimethylamino) ethoxyphosphonium ethylsulfate with 87% yield.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: acetone-d6, reference material: tetramethylsilane)
δ 4.47 4.38 (m, 2H)
3.86 (q, 2H)
2.90 (d, 18H)
1.45 (t, 3H)
1.13 (t, 3H)
The structural formula is shown below (a dashed line in the formula represents a conjugated structure).

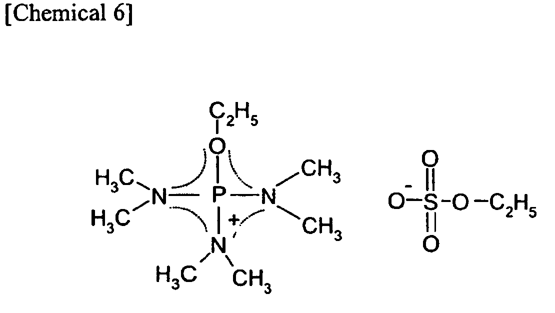

### (d) Preparation of tri(dimethylamino) ethoxyphosphonium bistrifluoromethane sulfonylimide

In 100 ml of pure water, 3.23 g (9.7 mmol) of tri(dimethylamino) ethoxyphosphonium ethylsulfate obtained in (c) were dissolved. After impurities were extracted with CH₂Cl₂, an aqueous solution dissolving 2.8 g (9.7 mmol) of lithium bistrifluoromethane sulfonylimide in 100 ml of pure water was added to the resulting aqueous solution while stirring. After 60-minute continuous stirring, the resulting hydrophobic white solid was washed with water two or three times, extracted with dichloromethane, and purified with an alumina column. The extract was concentrated, and then vacuum-dried at 80°C for 10 hours to obtain 4.35 g (yield: 92%) of a product compound that was a white solid at room temperature, and a colorless transparent liquid at 90°C.
The compound was identified with a nuclear magnetic resonance spectrometer (BRUKER Ultra Shield 300 NMR Spectrometer, supplied by BRUKER Corp.). The compound was identified as the objective compound of tri(dimethylamino) ethoxyphosphonium bistrifluoromethane sulfonylimide. The spectrum data are shown below.
¹H-NMR (300 MHz, solvent: acetone-d6, reference material: tetramethylsilane)
δ 4.46 4.37 (m, 2H)
2.90 (d, 18H)
1.45 (t, 3H)
¹⁹F-NMR (282 MHz, solvent: acetone-d6, reference material: CF₃Cl)
δ -79.91 (s, 6F)
The structural formula is shown below (a dashed line in the formula represents a conjugated structure).

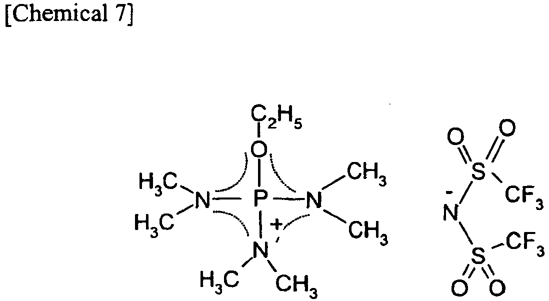

The melting point was measured with a scanning differential calorimeter (DSC8230, supplied by Shimadzu Corp.). The melting point was 88°C.

### Example 3

### (e) Preparation of tri(dimethylamino) butoxyphosphonium butylsulfate

In an eggplant-shaped two-neck flask quipped with a reflux condenser, a dropping funnel and a magnetic stirrer, 70.4 g (335 mmol) of dibutylsulfate were added dropwise to 50.0 g (279 mmol) of hexamethylphosphate triamide at room temperature in a nitrogen gas atmosphere. After 7-day stirring at 30°C, a white solid salt was obtained. The salt was washed sufficiently with ether and vacuum-dried at 50°C for 5 hours to obtain tri(dimethylamino) butoxyphosphonium butylsulfate with 93% yield.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: acetone-d6, reference material: tetramethylsilane)
δ 4.38 (q, 2H)
3.82 (t, 2H)
2.90 (d, 18H)
1.80-1.73 (m, 2H)
1.55-1.30 (m, 6H)
0.96 (t, 3H)
0.90 (t, 3H)
The structural formula is shown below (a dashed line in the formula represents a conjugated structure).

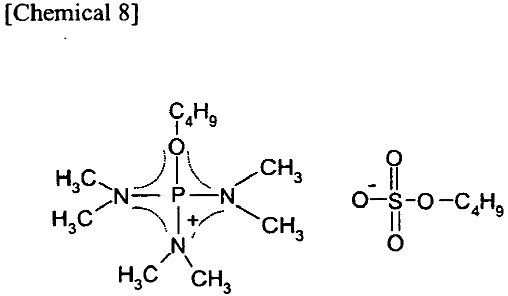

### (f) Preparation of tri(dimethylamino) butoxyphosphonium bistrifluoromethane sulfonylimide

In 200 ml of pure water, 58.4 g (150 mmol) of tri(dimethylamino) butoxyphosphonium butylsulfate obtained in (e) were dissolved. To the resulting aqueous solution, an aqueous solution dissolving 43.1 g (150 mmol) of lithium bistrifluoromethane sulfonylimide in 150 ml of pure water was added while stirring. After 2-hour continuous stirring, the resulting hydrophobic transparent liquid was washed with water five times and extracted with dichloromethane. The extract was concentrated, and then vacuum-dried at 80°C for 20 hours to obtain 76.9 g (yield: 99%) of a product compound that was a colorless transparent liquid at room temperature. The compound was identified with a nuclear magnetic resonance spectrometer (BRUKER Ultra Shield 300 NMR Spectrometer, supplied by BRUKER Corp.). The compound was identified as the objective compound of tri(dimethylamino) butoxyphosphonium bistrifluoromethane sulfonylimide. The spectrum data are shown below.
¹H-NMR (300 MHz, solvent: acetone-d6, reference material: tetramethylsilane)
δ 4.36 (q, 2H)
2.90 (d, 18H)
1.84-1.75 (m, 2H)
1.55-1.42 (m, 2H)
0.96 (t, 3H)
¹⁹F-NMR (282 MHz, solvent: acetone-d6, reference material: CF₃Cl)
δ -79.92 (s, 6F)
The structural formula is shown below (a dashed line in the formula represents a conjugated structure).

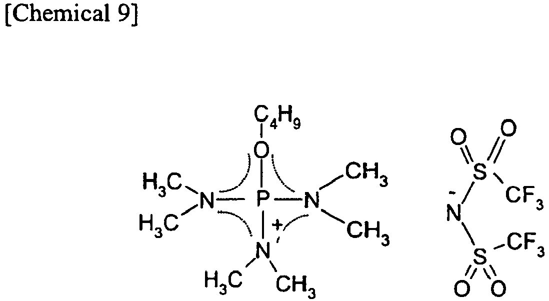

The melting point was measured with a scanning differential calorimeter (DSC8230, supplied by Shimadzu Corp.). The melting point was -7.5°C and the crystallization temperature was -67°C. The thermal decomposition temperature was measured with a thermogravimetric analyzer (TG8120, supplied by Rigaku Corp.). The weight loss starting temperature measured at a temperature elevation rate of 10°C/min was 200°C. These results show that the salt of the present example keeps a stable liquid state in a wide range of temperatures from -7.5°C to 200°C.
The viscosity measured with a vibration-type viscometer (supplied by A&D Co., Ltd.) was 45 mPa·s at 25°C.
The conductivity measured with the AC impedance method (Electrochemical Measurement System HZ-3000, supplied by Hokuto Denko Corp.) was 0.3 Sm⁻¹ at 25°C.
Further, the cyclic voltammogram measured with the Electrochemical Measurement System HZ-3000 supplied by Hokuto Denko Corp, using a Pt working electrode, a Pt counter electrode, and a Li reference electrode showed that the potential window was from -0.1 V to 4.9 V with reference to the Li/Li⁺ potential. The CV curve of tri(dimethylamino) butoxyphosphonium bistrifluoromethane sulfonylimide is shown in FIG. 1.

### Example 4

### (g) Preparation of tri(dimethylamino) butylphosphonium butylsulfate

In an eggplant-shaped two-neck flask equipped with a reflux condenser, a dropping funnel and a magnetic stirrer, 37.4 g (178 mmol) of diethylsulfate were added dropwise to 24.2 g (149 mmol) of hexamethylphosphorous triamide at room temperature in a nitrogen gas atmosphere. After 3-day stirring at room temperature, a white solid salt was obtained. The salt was washed sufficiently with ether and vacuum-dried at 50°C for 5 hours to obtain tri(dimethylamino) butylphosphonium butylsulfate with 94% yield.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: acetone-d6, reference material: tetramethylsilane)
δ 3.83 (t, 2H)
2.85 (d, 18H)
2.73-2.63 (m, 2H)
1.70-1.33 (m, 8H)
0.97 (t, 3H)
0.90 (t, 3H)
The structural formula is shown below (a dashed line in the formula represents a conjugated structure).

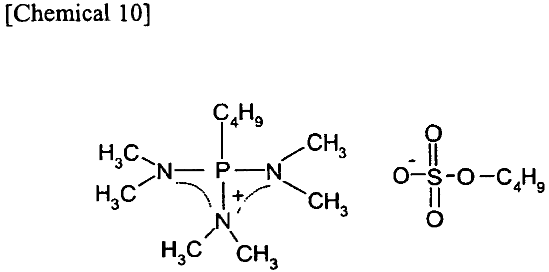

### (h) Preparation of tri(dimethylamino) butylphosphonium bistrifluoromethane sulfonylimide

In 200 ml of pure water, 37.4 g (100 mmol) of tri(dimethylamino) butylphosphonium butylsulfate obtained in (g) were dissolved. To the resulting aqueous solution, an aqueous solution dissolving 28.7 g (100 mmol) of lithium bistrifluoromethane sulfonylimide in 150 ml of pure water was added while stirring. After 2 hour-continuous stirring, the resulting hydrophobic transparent liquid was washed with pure water five times and extracted with dichloromethane. The extract was concentrated, and then vacuum-dried at 80°C for 20 hours to obtain 46.7 g (yield: 93%) of a product compound that was a colorless transparent liquid at room temperature.
The compound was identified with a nuclear magnetic resonance spectrometer (BRUKER Ultra Shield 300 NMR Spectrometer, supplied by BRUKER Corp.). The compound was identified as the objective compound of tri(dimethylamino) butylphosphonium bistrifluoromethane sulfonylimide. The spectrum data are shown below.
¹H-NMR (300 MHz, solvent: acetone-d6, reference material: tetramethylsilane)
δ 2.85 (d, 18H)
2.66-2.56 (m, 2H)
1.75-1.63 (m, 2H)
1.57-1.45 (m, 2H)
0.97 (t, 3H)
¹⁹F-NMR (282 MHz, solvent: acetone-d6, reference material: CF₃Cl)
δ -79.87 (s, 6F)
The structural formula is shown below (a dashed line in the formula represents a conjugated structure).

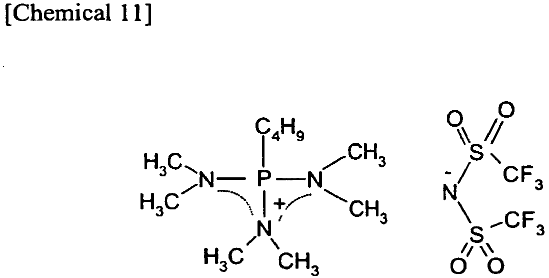

The melting point was measured with a scanning differential calorimeter (DSC8230, supplied by Shimadzu Corp.). The melting point was 20.8°C and the crystallization temperature was -0.6°C. The thermal decomposition temperature was measured with a thermogravimetric analyzer (TG8120, supplied by Rigaku Corp.). The weight loss starting temperature measured at a temperature elevation rate of 10°C/min was 320°C. These results show that the salt of the present example keeps a stable liquid state in a wide range of temperatures from 20.8°C to 320°C.
The viscosity measured with a vibration-type viscometer (supplied by A&D Co., Ltd.) was 53 mPa·s at 40°C.
The conductivity measured with the AC impedance method (Electrochemical Measurement System HZ-3000, supplied by Hokuto Denko Corp.) was 0.3 Sm⁻¹ at 40°C.
Further, the cyclic voltammogram measured with the Electrochemical Measurement System HZ-3000 supplied by Hokuto Denko Corp, using a Pt working electrode, a Pt counter electrode, and a Li reference electrode showed that the potential window was from 0 V to 4.9 V with reference to the Li/Li⁺ potential. The CV curve of tri(dimethylamino) butylphosphonium bistrifluoromethane sulfonylimide is shown in FIG. 2.

### Reference Example 5

### (i) Preparation of tris(methylbutylamino) phosphine

In a 1000 ml three-neck flask equipped with a dropping funnel and a magnetic stirrer, 8.7 ml (0.10 mol) of phosphorous trichloride and 1000 ml of anhydrous diethylether were added at room temperature in a nitrogen gas atmosphere. After the mixture was cooled in an ice bath, 70 ml (0.60 mol) of methylbutylamine were gradually added dropwise while stirring. After that, the reaction mixture was stirred for 1 hour with ice-cooling. The reaction mixture was filtered off under pressure in a nitrogen gas atmosphere, and the resulting crystals were washed with anhydrous diethylether three times. The crystals were purified by distillation at 105°C to 118°C under a reduced pressure of 0.2 kPa to obtain 21.28 tris(methylbutylamino) phosphine that was a transparent liquid. The yield was 74%.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: CDCl₃, reference material: tetramethylsilane)
δ 2.76 (m, 6H)
2.43 (d, 9H)
1.45 (m, 6H)
1.27 (m, 6H)
0.91 (t, 9H)
³¹P-NMR (121 MHz, solvent: CDCl₃, reference material: triphenylphosphine)
δ 120.88 (s, 1P)
The structural formula is shown below.

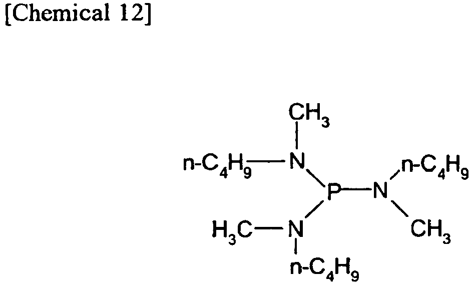

### (j) Preparation of tris(methylbutylamino) methylphosphonium methylsulfate

In a 50 ml two-neck flask equipped with a magnetic stirrer, 4.00 g (0.0138 mol) of tris(methylbutylamino) phosphine obtained in (i) were added at room temperature in a nitrogen gas atmosphere and ice-cooled, and then 1.6 ml (0.017 mol) of dimethylsulfate were added dropwise. After 12 hour-stirring at room temperature, the reaction mixture was washed with diethylether three times, and then vacuum-dried at room temperature to obtain 4.18 g of tris(methylbutylamino) methylphosphonium methylsulfate that was a transparent liquid at room temperature. The yield was 73%.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: CDCl₃, reference material: tetramethylsilane)
δ 3.71 (s, 3H)
2.96 (m, 6H)
2.76 (d, 9H)
2.09 (d, 3H)
1.57(m,6H)
1.33 (m, 6H)
0.96 (t, 9H)
³¹P-NMR (121 MHz, solvent: CDCl₃, reference material: triphenylphosphine)
δ 58.79 (m, 1P)
The structural formula is shown below (a dashed line in the formula represents a conjugated structure).

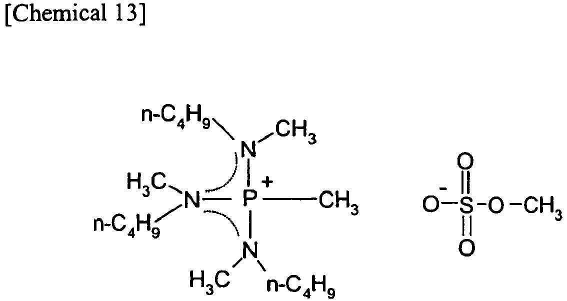

The melting point was measured with a scanning differential calorimeter (DSC8230, supplied by Shimadzu Corp.). The glass transition temperature was - 70.4°C. The thermal decomposition temperature was measured with a thermogravimetric analyzer (TG8120, supplied by Rigaku Corp.). The 5% weight loss temperature measured at a temperature elevation rate of 10°C/min was 263.5°C.

### Example 6

### (k) Preparation of tris(methylbutylamino) methylphosphonium bistrifluoromethane sulfonylimide

In a 100 ml eggplant-shaped flask equipped with a magnetic stirrer, 1.00 g (0.0024 mol) of tris(methylbutylamino) methylphosphonium methylsulfate obtained in (j) and 10 ml of ultrapure water were added, and then an aqueous solution dissolving 0.8 g (0.0026 mol) of lithium bistrifluoromethane sulfonylimide in 10 ml of ultrapure water was added while stirring. The reaction mixture was stirred at room temperature for 62 hours. The resulting salt was extracted with 20 ml of CH₂Cl₂, and the water layer was further extracted with 20 ml of CH₂Cl₂. After the organic layer was washed with 20 ml of ultrapure water three times, the extract was concentrated with a rotary evaporator, washed with diethylether three times, and then vacuum-dried at 80°C to obtain 0.91 g of tris(methylbutylamino) methylphosphonium bistrifluoromethane sulfonylimide that was a transparent liquid at room temperature. The yield was 65%.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: CDCl₃, reference material: tetramethylsilane)
δ 2.91 (m, 6H)
2.71 (d, 9H)
1.92 (d, 3H)
1.56 (m, 6H)
1.32 (m, 6H)
0.96 (t, 9H)
¹⁹F-NMR (282 MHz, solvent: CDCl₃, reference material: CF₃Cl)
δ -78.82 (s, 6F)
³¹P-NMR (121 MHz, solvent: CDCl₃, reference material: triphenylphosphine)
δ 57.98 (m, 1P)
The structural formula is shown below (a dashed line in the formula represents a conjugated structure).

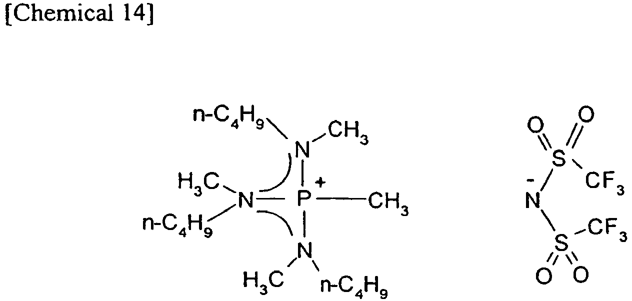

The melting point was measured with a scanning differential calorimeter (DSC8230, supplied by Shimadzu Corp.). The melting point was -5.5°C. The crystallization temperature was -48.4°C. The glass transition temperature was -82.9°C. The thermal decomposition temperature was measured with a thermogravimetric analyzer (TG8120, supplied by Rigaku Corp.). The 5% weight loss temperature measured at a temperature elevation rate of 10°C/min was 377.6°C.

### Example 7

### (1) Preparation of tris(methylbutylamino) methylphosphonium tetrafluoroborate

In a 100 ml eggplant-shaped flask equipped with a magnetic stirrer, 1.00 g (0.0024 mol) of tris(methylbutylamino) methylphosphonium methylsulfate obtained in (j) and 10 ml of ultrapure water were added, and then an aqueous solution dissolving 0.3 g (0.0026 mol) of ammonium tetrafluoroborate in 10 ml of ultrapure water was added while stirring. The reaction mixture was stirred at room temperature for 62 hours. The resulting salt was extracted with 20 ml of CH₂Cl₂, and the water layer was further extracted with 20 ml of CH₂Cl₂. After the organic layer was washed with 20 ml of ultrapure water three times, the extract was concentrated with a rotary evaporator, washed with diethylether three times, and then vacuum-dried at 80°C to obtain 0.60 g of tris(methylbutylamino) methylphosphonium tetrafluoroborate that was a white solid at room temperature. The yield was 64%.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: CDCl₃, reference material: tetramethylsilane)
δ 2.96 (m, 6H)
2.73 (d, 9H)
1.99 (d, 3H)
1.55 (m, 6H)
1.33 (m, 6H)
0.95 (t, 9H)
¹⁹F-NMR (282 MHz, solvent: CDCl₃, reference material: CF₃Cl)
δ -152.69 (d, 4F)
³¹P-NMR (121 MHz, solvent: CDCl₃, reference material: triphenylphosphine)
δ 58.72 (m, 1P)
The structural formula is shown below (a dashed line in the formula represents a conjugated structure).

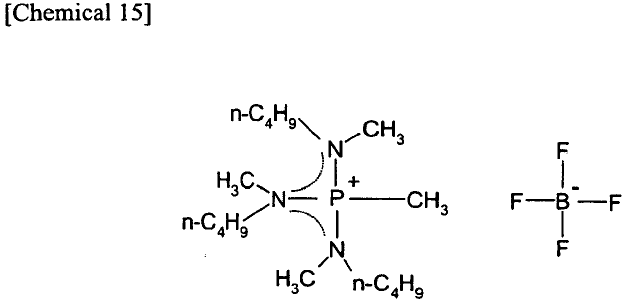

The melting point was measured with a scanning differential calorimeter (DSC8230, supplied by Shimadzu Corp.). The melting point was 116.5°C. The thermal decomposition temperature was measured with a thermogravimetric analyzer (TG8120, supplied by Rigaku Corp.). The 5% weight loss temperature measured at a temperature elevation rate of 10°C/min was 404.6°C.

### Example 8

### (m) Preparation of tris(methylbutylamino) methylphosphonium hexafluorophosphate

In a 100 ml eggplant-shaped flask equipped with a magnetic stirrer, 1.00 g (0.0024 mol) of tris(methylbutylamino) methylphosphonium methylsulfate obtained in (j) and 10 ml of ultrapure water were added, and then an aqueous solution dissolving 0.4 g (0.0026 mol) of lithium hexafluorophosphate in 10 ml of ultrapure water was added while stirring. The reaction mixture was stirred at room temperature for 86 hours. The resulting salt was extracted with 20 ml of CH₂Cl₂, and the water layer was further extracted with 20 ml of CH₂Cl₂. After the organic layer was washed with 20 ml of ultrapure water three times, the extract was concentrated with a rotary evaporator, washed with diethylether three times, and then vacuum-dried at 80°C to obtain 0.48 g of tris(methylbutylamino) methylphosphonium hexafluorophosphate that was a white solid at room temperature. The yield was 44%.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: CDCl₃, reference material: tetramethylsilane)
δ 2.92 (m, 6H)
2.72 (d, 9H)
1.92 (d, 3H)
1.56 (m, 6H)
1.32 (m, 6H)
0.96 (t, 9H)
¹⁹F-NMR (282 MHz, solvent: CDCl₃, reference material: CF₃Cl)
δ -72.84 (d, 6F)
³¹P-NMR (121 MHz, solvent: CDCl₃, reference material: triphenylphosphine)
δ 58.32 (m, 1P)
-144.25 (hept, 1P)
The structural formula is shown below (a dashed line in the formula represents a conjugated structure).

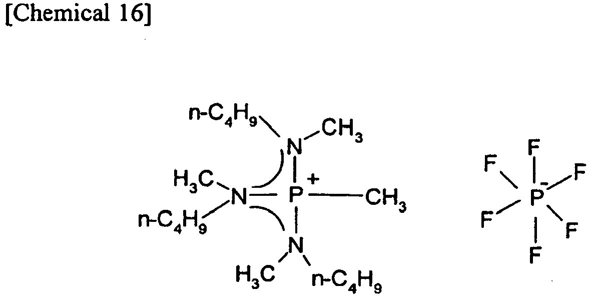

The melting point was measured with a scanning differential calorimeter (DSC8230, supplied by Shimadzu Corp.). No peak corresponding to the melting point was observed. The thermal decomposition temperature was measured with a thermogravimetric analyzer (TG8120, supplied by Rigaku Corp.). The 5% weight loss temperature measured at a temperature elevation rate of 10°C/min was 393.2°C.

### Reference Example 9

### (n) Preparation of tris(methylbutylamino) ethylphosphonium ethylsulfate

In a 50 ml two-neck flask equipped with a magnetic stirrer, 4.00 g (0.0138 mol) of tris(methylbutylamino) phosphine obtained in (i) were added at room temperature in a nitrogen gas atmosphere and ice-cooled, and then 2.2 ml (0.017 mol) of diethylsulfate were added dropwise. After 37-hour stirring at 30°C, the reaction mixture was washed with diethylether three times and vacuum-dried at room temperature to obtain 3.41 g of tris(methylbutylamino) ethylphosphonium ethylsulfate that was a transparent liquid at room temperature. The yield was 57%.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: CDCl₃, reference material: tetramethylsilane)
δ 4.09 (m, 2H)
2.96 (m, 6H)
2.78 (d, 9H)
2.60 (m, 2H)
1.59 (m, 6H)
1.40-1.24 (m, 12H)
0.96 (t, 9H)
³¹P-NMR (121 MHz, solvent: CDCl₃, reference material: triphenylphosphine)
δ 61.87 (m, 1P)
The structural formula is shown below (a dashed line in the formula represents a conjugated structure).

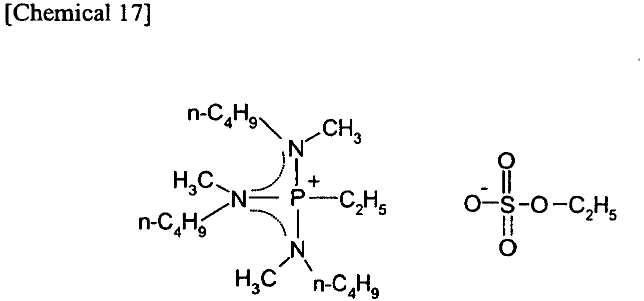

The melting point was measured with a scanning differential calorimeter (DSC8230, supplied by Shimadzu Corp.). No peak corresponding to the melting point was observed. The thermal decomposition temperature was measured with a thermogravimetric analyzer (TG8120, supplied by Rigaku Corp.). The 5% weight loss temperature measured at a temperature elevation rate of 10°C/min was 250.5°C.

### Example 10

### (o) Preparation of tris(methylbutylamino) ethylphosphonium bistrifluoromethane sulfonylimide

In a 100 ml eggplant-shaped flask equipped with a magnetic stirrer, 1.00 g (0.0023 mol) of tris(methylbutylamino) ethylphosphonium ethylsulfate obtained in (n) and 10 ml of ultrapure water were added, and then an aqueous solution dissolving 0.8 g (0.0026 mol) of lithium bistrifluoromethane sulfonylimide in 10 ml of ultrapure water was added while stirring. The reaction mixture was stirred at room temperature for 62 hours. The resulting salt was extracted with 20 ml of CH₂Cl₂, and the water layer was further extracted with 20 ml of CH₂Cl₂. After the organic layer was washed with 20 ml of ultrapure water three times, the extract was concentrated with a rotary evaporator, washed with diethylether three times, and then vacuum-dried at 80°C to obtain 0.73 g of tris(methylbutylamino) ethylphosphonium bistrifluoromethane sulfonylimide that was a transparent liquid at room temperature. The yield was 53%.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: CDCl₃, reference material: tetramethylsilane)
δ 2.92 (m, 6H)
2.72 (d, 9H)
2.37 (m, 2H)
1.58 (m, 6H)
1.39-1.20 (m, 9H)
0.97 (t, 9H)
¹⁹F-NMR (282 MHz, solvent: CDCl₃, reference material: CF₃Cl)
δ -78.83 (s, 6F)
³¹P-NMR (121 MHz, solvent: CDCl₃, reference material: triphenylphosphine) δ 61.02 (m, 1P)
The structural formula is shown below (a dashed line in the formula represents a conjugated structure).

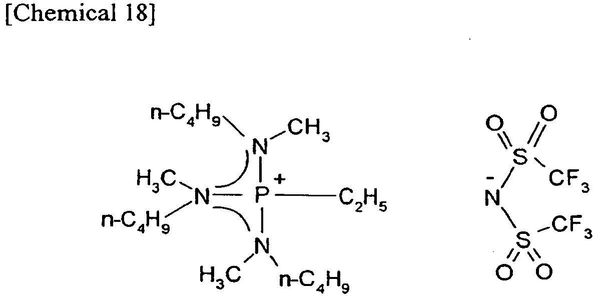

The melting point was measured with a scanning differential calorimeter (DSC8230, supplied by Shimadzu Corp.). The melting point was -20.6°C. The glass transition temperature was -84.6°C. The thermal decomposition temperature was measured with a thermogravimetric analyzer (TG8120, supplied by Rigaku Corp.). The 5% weight loss temperature measured at a temperature elevation rate 10°C/min was 362.8°C.
The conductivity measured with the AC impedance method (Electrochemical Measurement System HZ-3000, supplied by Hokuto Denko Corp.) was 0.085 Sm⁻¹ at 25°C.

### Example 11

### (p) Preparation of tris(methylbutylamino) ethylphosphonium tetrafluoroborate

In a 100 ml eggplant-shaped flask equipped with a magnetic stirrer, 0.86 g (0.0019 mol) of tris(methylbutylamino) ethylphosphonium ethylsulfate obtained in (n) and 10 ml of ultrapure water were added, and then an aqueous solution dissolving 0.3 g (0.0026 mol) of ammonium tetrafluoroborate in 10 ml of ultrapure water was added while stirring. The reaction mixture was stirred at room temperature for 14 hours. The resulting salt was extracted with 20 ml of CH₂Cl₂, and the water layer was further extracted with 20 ml of CH₂Cl₂. After the organic layer was washed with 20 ml of ultrapure water three times, the extract was concentrated with a rotary evaporator, washed with diethylether three times, and then vacuum-dried at 80°C to obtain 0.65 g of tris(methylbutylamino) ethylphosphonium tetrafluoroborate that was a transparent liquid at room temperature. The yield was 84%.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: CDCl₃, reference material: tetramethylsilane)
δ 2.95 (m, 6H)
2.75 (d, 9H)
2.45 (m, 2H)
1.58 (m, 6H)
1.37-1.22 (m, 9H)
0.96 (t, 9H)
¹⁹F-NMR (282 MHz, solvent: CDCl₃, reference material: CF₃Cl)
δ -153.27 (d, 4F)
³¹P-NMR (121 MHz, solvent: CDCl₃, reference material: triphenylphosphine) δ 61.41 (m, 1P)
The structural formula is shown below (a dashed line in the formula represents a conjugated structure).

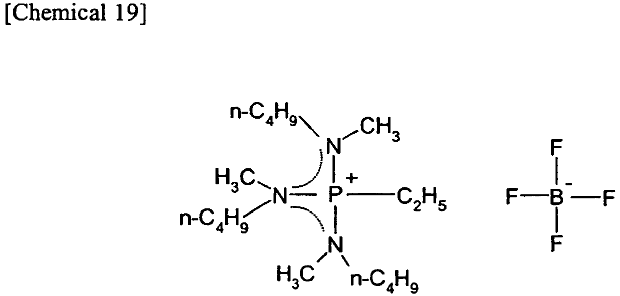

The melting point was measured with a scanning differential calorimeter (DSC8230, supplied by Shimadzu Corp.). The melting point was 1.0°C. The crystallization temperature was -32.7°C. The glass transition temperature was - 75.5°C. The thermal decomposition temperature was measured with a thermogravimetric analyzer (TG8120, supplied by Rigaku Corp.). The 5% weight loss temperature measured at a temperature elevation rate of 10°C/min was 389.1°C.

### Example 12

### (q) Preparation of tris(methylbutylamino) ethylphosphonium hexafluorophosphate

In a 100 ml eggplant-shaped flask equipped with a magnetic stirrer, 1.00 g (0.0023 mol) of tris(methylbutylamino) ethylphosphonium ethylsulfate obtained in (n) and 10 ml of ultrapure water were added, and then an aqueous solution dissolving 0.7 g (0.0046 mol) of lithium hexafluorophosphate in 10 ml of ultrapure water was added while stirring. The reaction mixture was stirred at room temperature for 14 hours. The resulting salt was extracted with 20 ml of CH₂Cl₂, and the water layer was further extracted with 20 ml of CH₂Cl₂. After the organic layer was washed with 20 ml of ultrapure water three times, the extract was concentrated with a rotary evaporator, washed with diethylether three times, and then vacuum-dried at 80°C to obtain 0.65 g of tris(methylbutylamino) ethylphosphonium hexafluorophosphate that was a transparent liquid at room temperature. The yield was 44%.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: CDCl₃, reference material: tetramethylsilane)
δ 2.93 (m, 6H)
2.73 (d, 9H)
2.47 (m, 2H)
1.58 (m, 6H)
1.37-1.20 (m, 9H)
0.95 (t, 9H)
¹⁹F-NMR (282 MHz, solvent: CDCl₃, reference material: CF₃Cl)
δ -73.15 (d, 6F)
³¹P-NMR (121 MHz, solvent: CDCl₃, reference material: triphenylphosphine) δ 61.00 (m, 1P)
-144.29 (hept, 1P)
The structural formula is shown below (a dashed line in the formula represents a conjugated structure).

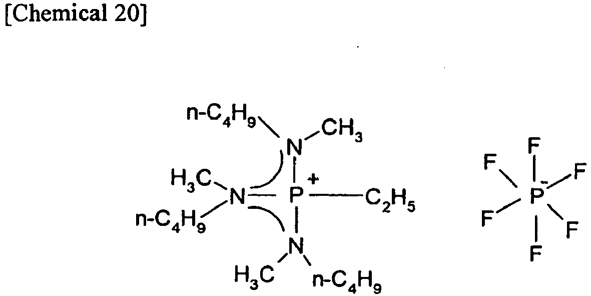

The melting point was measured with a scanning differential calorimeter (DSC8230, supplied by Shimadzu Corp.). No peak corresponding to the melting point was observed. The thermal decomposition temperature was measured with a thermogravimetric analyzer (TG8120, supplied by Rigaku Corp.). The 5% weight loss temperature measured at a temperature elevation rate of 10°C/min was 319.5°C.

### Reference Example 13

### (r) Preparation of tris(methylethylamino) n-butylphosphonium n-butylsulfate

In a 50 ml two-neck flask equipped with a magnetic stirrer, 2.33 g (0.0114 mol) of tris(methylethylamino) phosphine obtained similarly to (i) were added at room temperature in a nitrogen gas atmosphere. After ice-cooling, 2.7 ml (0.0136 mol) of di-n-butylsulfate were added dropwise. The reaction mixture was stirred for 87 hours at room temperature, and then for 72 hours at 30°C. After that, the reaction mixture was washed with diethylether three times, and vacuum-dried at room temperature so as to obtain 3.83 g of tris(methylethylamino) n-butylphosphonium n-butylsulfate that was a transparent liquid at room temperature. The yield was 94%.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: CDCl₃, reference material: tetramethylsilane)
δ 4.04 (t,2H)
3.11 (m, 6H)
2.77 (d, 9H)
2.48 (m, 2H)
1.67-1.37 (m, 8H)
1.24 (t, 9H)
0.99-0.88 (m, 6H)
³¹P-NMR (121 MHz, solvent: CDCl₃, reference material: triphenylphosphine)
δ 59.52 (m, 1P)
The structural formula is shown below (a dashed line in the formula represents a conjugated structure).

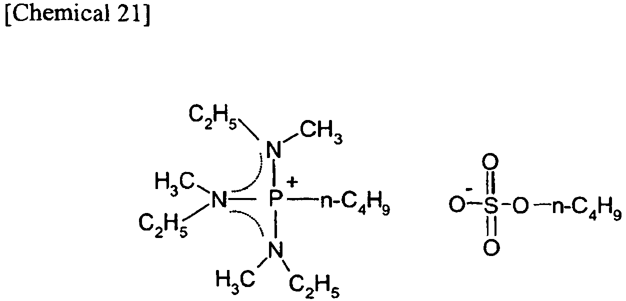

### Example 14

### (s) Preparation of tris(methylethylamino) n-butylphosphonium bistrifluoromethane sulfonylimide

In a 100 ml eggplant-shaped flask equipped with a magnetic stirrer, 1.00 g (0.0024 mol) of tris(methylethylamino) n-butylphosphonium n-butylsulfate obtained in (r) and 10 ml of ultrapure water were added, and then an aqueous solution dissolving 0.9 g (0.0029 mol) of lithium bistrifluoromethane sulfonylimide in 10 ml of ultrapure water was added while stirring. The reaction mixture was stirred at room temperature for 14 hours. The resulting salt was extracted with 20 ml of CH₂Cl₂, and the water layer was further extracted with 20 ml of CH₂Cl₂. After the organic layer was washed with 20 ml of ultrapure water three times, the extract was concentrated with a rotary evaporator, washed with diethylether three times, and then vacuum-dried at 80°C to obtain 0.74 g of tris(methylethylamino) n-butylphosphonium bistrifluoromethane sulfonylimide that was a transparent liquid at room temperature. The yield was 57%.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: CDCl₃, reference material: tetramethylsilane)
δ 3.05 (m, 6H)
2.72 (d, 9H)
2.28 (m, 2H)
1.51 (m, 4H)
1.23 (t, 9H)
0.97 (t, 3H)
¹⁹F-NMR (282 MHz, solvent: CDCl₃, reference material: CF₃Cl)
δ -78.84 (s, 6F)
³¹P-NMR (121 MHz, solvent: CDCl₃, reference material: triphenylphosphine)
δ 59.02 (m, 1P)
The structural formula is shown below (a dashed line in the formula represents a conjugated structure).

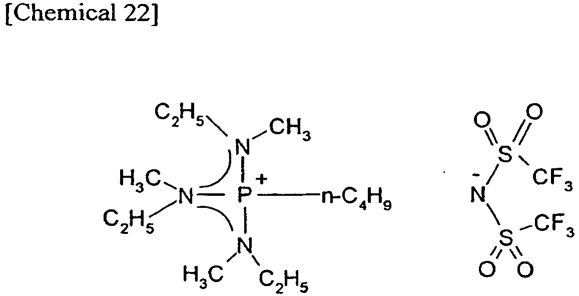

The melting point was measured with a scanning differential calorimeter (DSC8230, supplied by Shimadzu Corp.). The melting point was -18.7°C. The crystallization temperature was -47.9°C. The thermal decomposition temperature was measured with a thermogravimetric analyzer (TG8120, supplied by Rigaku Corp.). The 5% weight loss temperature measured at a temperature elevation rate of 10°C/min was 393.0°C.

### Example 15

### (t) Preparation of tris(methylethylamino) n-butylphosphonium tetrafluoroborate

In a 100 ml eggplant-shaped flask equipped with a magnetic stirrer, 1.00 g (0.0024 mol) of tris(methylethylamino) n-butylphosphonium n-butylsulfate obtained in (r) and 10 ml of ultrapure water were added, and then an aqueous solution dissolving 0.4 g (0.0029 mol) of ammonium tetrafluoroborate in 10 ml of ultrapure water was added while stirring. The reaction mixture was stirred at room temperature for 14 hours. The resulting salt was extracted with 20 ml of CH₂Cl₂, and the water layer was further extracted with 20 ml of CH₂Cl₂. After the organic layer was washed with 20 ml of ultrapure water three times, the extract was concentrated with a rotary evaporator, washed with diethylether three times, and then vacuum-dried at 80°C to obtain 0.87 g of tris(methylethylamino) n-butylphosphonium tetrafluoroborate that was a white solid at room temperature. The yield was 99%.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: CDCl₃, reference material: tetramethylsilane)
δ 3.08 (m, 6H)
2.75 (d, 9H)
2.38 (m, 2H)
1.53 (m, 4H)
1.23 (t, 9H)
0.97 (t, 3H)
¹⁹F-NMR (282 MHz, solvent: CDCl₃, reference material: CF₃Cl)
δ -153.07 (d, 4F)
³¹P-NMR (121 MHz, solvent: CDCl₃, reference material: triphenylphosphine) δ 59.40 (m, 1P)
The structural formula is shown below (a dashed line in the formula represents a conjugated structure).

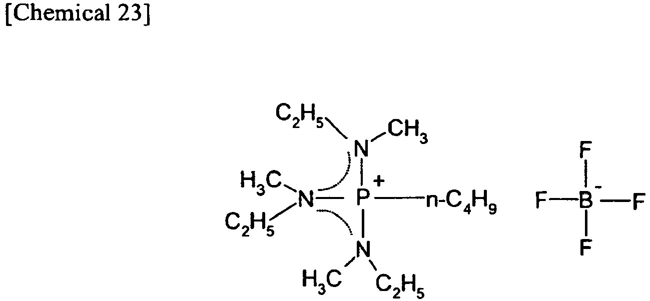

The melting point was measured with a scanning differential calorimeter (DSC8230, supplied by Shimadzu Corp.). No peak corresponding to the melting point was observed. The thermal decomposition temperature was measured with a thermogravimetric analyzer (TG8120, supplied by Rigaku Corp.). The 5% weight loss temperature measured at a temperature elevation rate of 10°C/min was 333.0°C.

### Example 16

### (u) Preparation of tris(methylethylamino) n-butylphosphonium hexafluorophosphate

In a 100 ml eggplant-shaped flask equipped with a magnetic stirrer, 1.00 g (0.0024 mol) of tris(methylethylamino) n-butylphosphonium n-butylsulfate obtained in (r) and 10 ml of ultrapure water were added, and then an aqueous solution dissolving 0.5 g (0.0029 mol) of lithium hexafluorophosphate in 10 ml of ultrapure water was added while stirring. The reaction mixture was stirred at room temperature for 14 hours. The resulting salt was extracted with 20 ml of CH₂Cl₂, and the water layer was further extracted with 20 ml of CH₂Cl₂. After the organic layer was washed with 20 ml of ultrapure water three times, the extract was concentrated with a rotary evaporator, washed with diethylether three times, and then vacuum-dried at 80°C to obtain 0.95 g of tris(methylethylamino) n-butylphosphonium hexafluorophosphate that was a white solid at room temperature. The yield was 97%.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: CDCl₃, reference material: tetramethylsilane)
δ 3.06 (m, 6H)
2.72 (d, 9H)
2.3 9 (m, 2H)
1.52(m,4H)
1.22 (t, 9H)
0.97 (t, 3H)
¹⁹F-NMR (282 MHz, solvent: CDCl₃, reference material: CF₃Cl)
δ -73.08 (d, 6F)_{.}
³¹P-NMR (121 MHz, solvent: CDCl₃, reference material: triphenylphosphine) δ 59.08 (m, 1P)
-144.27 (hept, 1P)
The structural formula is shown below (a dashed line in the formula represents a conjugated structure).

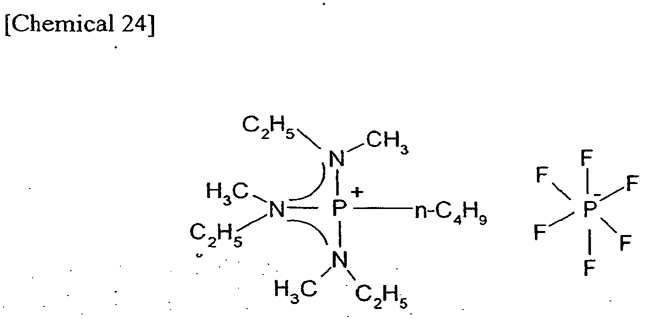

The melting point was measured with a scanning differential calorimeter (DSC8230, supplied by Shimadzu Corp.). No peak corresponding to the melting point was observed. The thermal decomposition temperature was measured with a thermogravimetric analyzer (TG8120, supplied by Rigaku Corp.). The 5% weight loss temperature measured at a temperature elevation rate of 10°C/min was 369.2°C.

### Reference Example 17

### (v) Preparation of tris(methylbutylamino) phosphine oxide

In a 200 ml three-neck flask equipped with a dropping funnel and a magnetic stirrer, 1.8 ml (0.020 mol) of phosphoryl chloride and 100 ml of dehydrated dibutylether were added at room temperature in a nitrogen gas atmosphere. After the mixture was cooled in an ice bath, 21 ml (0.180 mol) of methylbutylamine were gradually added dropwise while stirring. The reaction mixture was further stirred at 120°C for 36 hours, and then the reaction mixture was filtered under pressure in a nitrogen gas atmosphere. The resulting crystals were washed with dehydrated dibutylether three times, and purified by distillation under a reduced pressure of 0.2 kPa at a temperature of 119 to 124°C so as to obtain 5.54 g of tris(methylbutylamino) oxoline, that was a transparent liquid. The yield was 74%.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: CDCl₃, reference material: tetramethylsilane)
δ 2.94 (m, 6H)
2.66 (d, 9H)
1.51 (m, 6H)
1.30 (m, 6H)
0.93 (t, 9H)
³¹P-NMR (121 MHz, solvent: CDCl₃, reference material: triphenylphosphine)
δ 25.26 (m, 1P)
The structural formula is shown below.

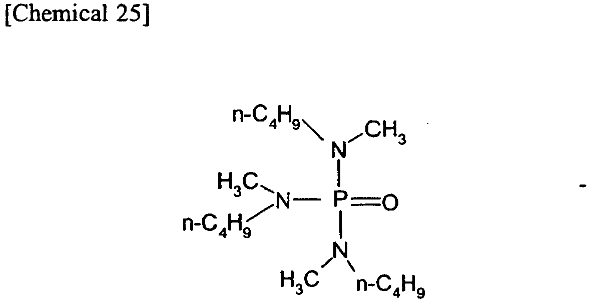

### (w) Preparation of tris(methylbutylamino) ethoxyphosphonium ethylsulfate

In a 50 ml two-neck flask equipped with a magnetic stirrer, 2.26 g (0.0074 mol) of tris(methylbutylamino) oxoline obtained in (v) were added at room temperature in a nitrogen gas atmosphere, and then 1.2 ml (0.0089 mol) of diethylsulfate were added dropwise. The mixture was stirred at 30°C for 69 hours, and then washed with diethylether three times and vacuum-dried at room temperature so as to obtain 0.65 g of tris(methylbutylamino) ethoxyphosphonium ethylsulfate that was a transparent liquid at room temperature. The yield was 19%.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: CDCl₃, reference material: tetramethylsilane)
δ 4.36 (m, 2H)
4.10 (q, 2H)
3.02 (m, 6H)
2.84 (d, 9H)
1.58 (m, 6H)
1.45 (t, 3H)
1.40-1.26 (m, 9H)
0.96 (t, 9H)
³¹P-NMR (121 MHz, solvent: CDCl₃, reference material: triphenylphosphine) δ 35.87 (m, 1P)
The structural formula is shown below (a dashed line in the formula represents a conjugated structure).

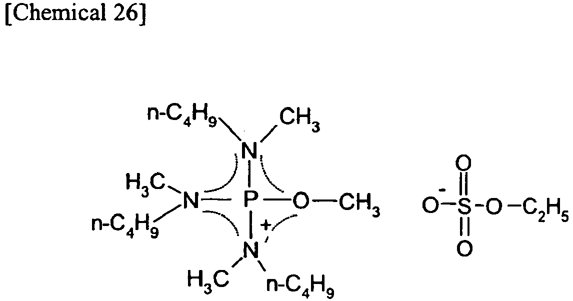

### Example 18

### (x) Preparation of tris(methylbutylamino) ethoxyphosphonium bistrifluoromethane sulfonylimide

In a 50 ml eggplant-shaped flask equipped with a magnetic stirrer, 0.65 g (0.0014 mol) of tris(methylbutylamino) ethoxyphosphonium ethylsulfate obtained in (w) and 10 ml of ultrapure water were added, and then an aqueous solution dissolving 0.5 g (0.0015 mol) of lithium bistrifluoromethane sulfonylimide in 10 ml of ultrapure water was added while stirring. The reaction mixture was stirred at 30°C for 62 hours. The resulting salt was extracted with 20 ml of CH₂Cl₂, and the water layer was further extracted with 20 ml of CH₂Cl₂. After the organic layer was washed with 40 ml of ultrapure water three times, the extract was concentrated with a rotary evaporator, washed with diethylether three times, and then vacuum-dried at 80°C to obtain 0.8 g of tris(methylbutylamino) ethoxyphosphonium bistrifluoromethane sulfonylimide that was a transparent liquid at room temperature. The yield was 93%.
The resulting compound was identified with a nuclear magnetic resonance spectrometer (BRUKER Ultra Shield 300 NMR Spectrometer, supplied by BRUKER Corp.). The spectrum data are shown below.
¹H-NMR (300 MHz, solvent: CDCl₃, reference material: tetramethylsilane)
δ 4.23 (m, 2H)
2.98 (m, 6H)
2.77 (d, 9H)
1.58 (m, 6H)
1.46-1.27 (m, 9H)
0.96 (t, 9H)
¹⁹F-NMR (282 MHz, solvent: CDCl₃, reference material: CF₃Cl)
δ - 78.83 (s, 6F)
³¹P-NMR (121 MHz, solvent: CDCl₃, reference material: triphenylphosphine)
δ 35.83 (m, 1P)
The structural formula is shown below (a dashed line in the formula represents a conjugated structure).

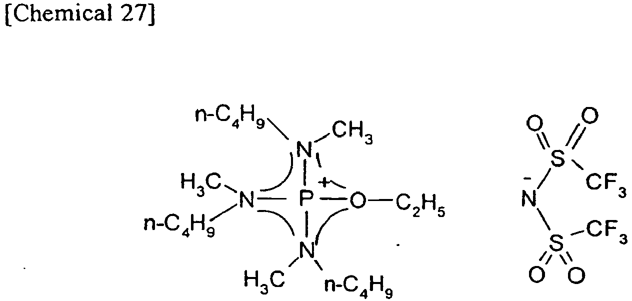

The melting point was measured with a scanning differential calorimeter (DSC8230, supplied by Shimadzu Corp.). The melting point was -19.9°C. The crystallization temperature was -55.8°C. The glass transition temperature was -85.9°C. The thermal decomposition temperature was measured with a thermogravimetric analyzer (TG8120, supplied by Rigaku Corp.). The 5% weight loss temperature measured at a temperature elevation rate of 10°C/min was 208.6°C.

### Industrial Applicability

According to the present invention, an ionic liquid that exhibits a stable liquid state in a wide range of temperatures from low temperatures, and has a low viscosity, an adequate conductivity and an excellent electrochemical stability, can be provided.
The ionic liquid of the present invention can be used for applications such as rechargeable lithium batteries; electrical double layer capacitors; fuel cells; dye sensitized solar cells; electrolytes, electrolyte solutions or additives for electric storage devices; reaction solvents; and the like.

## Claims

1. An ionic liquid comprising a cation component and an anion component, the cation component being one or plural kinds selected from the group consisting of cation components represented by the following formula (1): 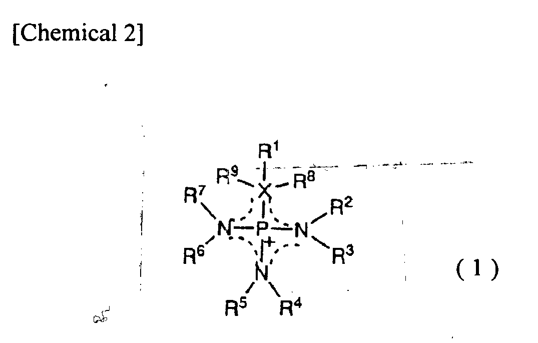 wherein substitution groups R¹ to R⁹ may be independently the same or different from one another; X represents a carbon atom or an oxygen atom; R⁸ and R⁹ exist only when X is a carbon atom; and a dashed line represents a conjugated structure,
wherein, if X is a carbon atom, each of R² to R⁷ is a straight chain alkyl group having 1 to 4 carbon atoms; each of R⁸ and R⁹ is a hydrogen atom; and R' is a straight chain or branched chain alkyl group having 1 to 10 carbon atoms or a straight chain or branched chain alkoxy group having 1 to 10 carbon atoms; and the anion component is (CF₃SO₂)₂N⁻, PF₆ or BF₄⁻; or
wherein, if X is an oxygen atom, each of R¹ to R⁷ is a straight chain or branched chain alkyl group having 1 to 10 carbon atoms, and may be the same or different from one another; and the anion component is one or plural kinds selected from the group consisting of [R^{f}SO₃]⁻, [(R^{f}SO₂)₂N]⁻, CF₃SO₃⁻, CF₃COO⁻, PF₆⁻, BF₄⁻, [N(CN)₂]⁻, [AlCl₄]⁻, SO₄²⁻, HSO₄⁻, NO₃⁻, F⁻, Br⁻ and I⁻; wherein R^{f} is a fluorine-containing substitution group.

2. The ionic liquid according to claim 1, wherein in the general formula (1), X is an oxygen atom, each of R² to R⁷ is a straight chain alkyl group having 1 to 4 carbon atoms; R¹ is a straight chain or branched chain alkyl group having 1 to 10 carbon atoms ; and the anion component is (CF₃SO₂)₂N⁻, PF₆ or BF₄⁻.

3. The ionic liquid according to claim 1, wherein in the general formula (1), X is a carbon atom, each of R² to R⁷ is a straight chain alkyl group having 1 to 4 carbon atoms; each of R⁸ and R⁹ is a hydrogen atom; and R¹ is a straight chain or branched chain alkyl group having 1 to 10 carbon atoms or a straight chain or branched chain alkoxy group having 1 to 10 carbon atoms; and the anion component is (CF₃SO₂)₂N⁻or PF₆⁻.

4. An electric storage device comprising the ionic liquid according to any of claims 1 to 3 as an electrolyte solution.

5. A rechargeable lithium battery comprising the ionic liquid according to any of claims 1 to 3.

6. An electrical double layer capacitor comprising the ionic liquid according to any of claims 1 to 3.

7. A dye sensitized solar cell comprising the ionic liquid according to any of claims 1 to 3.

8. A fuel cell comprising the ionic liquid according any of claims 1 to 3.

9. A reaction solvent comprising the ionic liquid according to any of claims 1 to 3.

10. A method for producing an ionic liquid comprising alkylating an organic substance represented by the following general formula (2),
the ionic liquid containing an organic substance represented by the general formula (1) as a cation component, 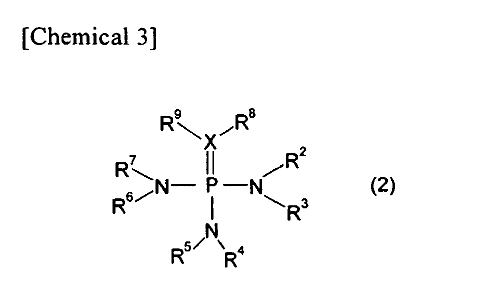 wherein substitution groups R² to R⁹ may be independently the same or different from one another; X represents a carbon atom or an oxygen atom; and R⁸ and R⁹ exist only when X is a carbon atom,
wherein, if X is a carbon atom, each of R² to R⁷ is a straight chain alkyl group having 1 to 4 carbon atoms; each of R⁸ and R⁹ is a hydrogen atom; and R¹ is a straight chain or branched chain alkyl group having 1 to 10 carbon atoms or a straight chain or branched chain alkoxy group having 1 to 10 carbon atoms; or
wherein, if X is an oxygen atom, each of R² to R⁷ is a straight chain or branched chain alkyl group having 1 to 10 carbon atoms, and may be the same or different from one another.

11. The method for producing an ionic liquid according to claim 10 comprising anion exchange with a resulting salt after the alkylation.

## Patentansprüche

1. Ionische Flüssigkeit, umfassend eine Kationenkomponente und eine Anionenkomponente, wobei die Kationenkomponente eine oder mehrere Arten ist bzw. sind, die aus der Gruppe ausgewählt werden, die aus Kationenkomponenten besteht, die durch die folgende Formel (1) dargestellt werden: 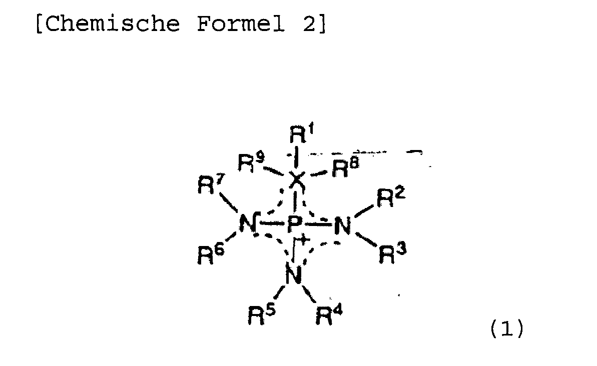 wobei die Substitutionsgruppen R¹ bis R⁹ unabhängig voneinander die gleichen sein können oder voneinander verschieden sein können; X ein Kohlenstoffatom oder ein Sauerstoffatom darstellt; R⁸ und R⁹ nur existieren, wenn X ein Kohlenstoffatom ist; und eine gestrichelte Linie eine konjugierte Struktur darstellt,
wobei, falls X ein Kohlenstoffatom ist, jede von R² bis R⁷ eine geradkettige Alkylgruppe ist, die 1 bis 4 Kohlenstoffatome aufweist; jede von R⁸ und R⁹ ein Wasserstoffatom ist; und R¹ eine geradkettige oder verzweigtkettige Alkylgruppe, die 1 bis 10 Kohlenstoffatome aufweist, oder eine geradkettige oder verzweigtkettige Alkoxygruppe, die 1 bis 10 Kohlenstoffatome aufweist, ist; und die Anionenkomponente (CF₃SO₂)₂N⁻, PF₆⁻ oder BF₄⁻ ist; oder
wobei, falls X ein Sauerstoffatom ist, jede von R¹ bis R⁷ eine geradkettige oder verzweigtkettige Alkylgruppe ist, die 1 bis 10 Kohlenstoffatome aufweist, und die die gleichen oder voneinander verschieden sein können; und die Anionenkomponente eine oder mehrere Arten ist bzw. sind, die aus der Gruppe ausgewählt werden, die aus [R^{f}SO₃]⁻, [(RfSO₂)₂N]⁻, CF₃SO₃⁻, CF₃COO⁻, PF₆⁻, BF₄⁻, [N(CN)₂]⁻, [AlCl₄]⁻, SO₄²⁻, HSO₄⁻, NO₃⁻, F⁻, Br⁻, Cl⁻, und I⁻ besteht; wobei R^{f} eine Fluor-enthaltende Substitutionsgruppe ist.

2. Ionische Flüssigkeit nach Anspruch 1, wobei in der allgemeinen Formel (1) X ein Sauerstoffatom ist, jede von R² bis R⁷ eine geradkettige Alkylgruppe ist, die 1 bis 4 Kohlenstoffatome aufweist; R¹ eine geradkettige oder verzweigtkettige Alkylgruppe ist, die 1 bis 10 Kohlenstoffatome aufweist; und die Anionenkomponente (CF₃SO₂)₂N⁻, PF₆⁻ oder BF₄⁻ ist.

3. Ionische Flüssigkeit nach Anspruch 1, wobei in der allgemeinen Formel (1) X ein Kohlenstoffatom ist, jede von R² bis R⁷ eine geradkettige Alkylgruppe ist, die 1 bis 4 Kohlenstoffatome aufweist; jede von R⁸ und R⁹ ein Wasserstoffatom ist; und R¹ eine geradkettige oder verzweigtkettige Alkylgruppe, die 1 bis 10 Kohlenstoffatome aufweist, oder eine geradkettige oder verzweigtkettige Alkoxygruppe, die 1 bis 10 Kohlenstoffatome aufweist, ist; und die Anionenkomponente (CF₃SO₂)₂N⁻ oder PF₆⁻ ist.

4. Elektrische Speichereinheit, umfassend die ionische Flüssigkeit nach einem der Ansprüche 1 bis 3 als eine Elektrolytlösung.

5. Wiederaufladbare Lithiumbatterie, umfassend die ionische Flüssigkeit nach einem der Ansprüche 1 bis 3.

6. Elektrischer Doppelschicht-Kapazitor, umfassend die ionische Flüssigkeit nach einem der Ansprüche 1 bis 3.

7. Farbstoff-sensibilisierte Solarzelle, umfassend die ionische Flüssigkeit nach einem der Ansprüche 1 bis 3.

8. Brennstoffzelle, umfassend die ionische Flüssigkeit nach einem der Ansprüche 1 bis 3.

9. Reaktionslösungsmittel, umfassend die ionische Flüssigkeit nach einem der Ansprüche 1 bis 3.

10. Verfahren zum Herstellen einer ionischen Flüssigkeit, umfassend das Alkylieren einer organischen Substanz, die durch die folgende allgemeine Formel (2) dargestellt ist,
wobei die ionische Flüssigkeit eine organische Substanz, die durch die allgemeine Formel (1) dargestellt ist, als eine Kationenkomponente enthält: 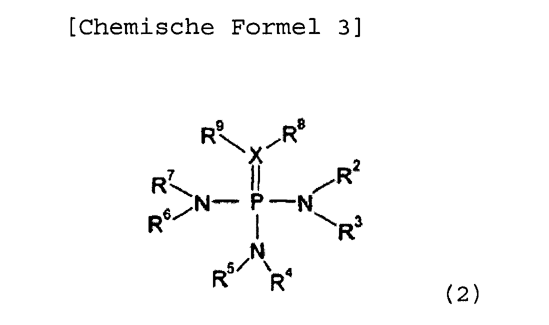 wobei die Substitutionsgruppen R² bis R⁹ unabhängig voneinander die gleichen sein können oder voneinander verschieden sein können; X ein Kohlenstoffatom oder ein Sauerstoffatom darstellt; und R⁸ und R⁹ nur existieren, wenn X ein Kohlenstoffatom ist,
wobei, falls X ein Kohlenstoffatom ist, jede von R² bis R⁷ eine geradkettige Alkylgruppe ist, die 1 bis 4 Kohlenstoffatome aufweist; jede von R⁸ und R⁹ ein Wasserstoffatom ist; und R¹ eine geradkettige oder verzweigtkettige Alkylgruppe, die 1 bis 10 Kohlenstoffatome aufweist, oder eine geradkettige oder verzweigtkettige Alkoxygruppe, die 1 bis 10 Kohlenstoffatome aufweist, ist; oder
wobei, falls X ein Sauerstoffatom ist, jede von R² bis R⁷ eine geradkettige oder verzweigtkettige Alkylgruppe ist, die 1 bis 10 Kohlenstoffatome aufweist, und die die gleichen oder voneinander verschieden sein können.

11. Verfahren zum Herstellen einer ionischen Flüssigkeiten nach Anspruch 10, umfassend eine Anionenaustausch mit einem resultierenden Salz nach der Alkylierung.

## Revendications

1. Liquide ionique comprenant un composant cationique et un composant anionique, le composant cationique étant un ou plusieurs type(s) choisi(s) dans le groupe constitué de composants cationiques représentés par la formule suivante (1) : 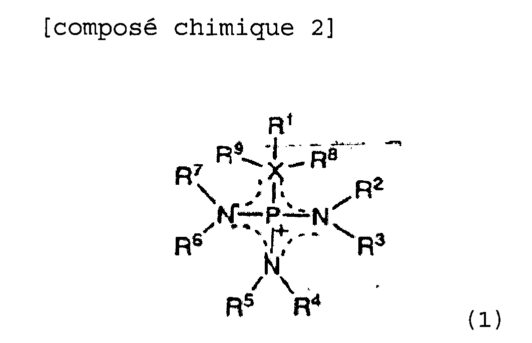 où des groupes de substitution R¹ à R⁹ peuvent être indépendamment identiques ou différents les uns des autres; X représente un atome de carbone ou un atome d'oxygène; R⁸ et R⁹ existent seulement lorsque X est un atome de carbone; et une ligne en tireté représente une structure conjuguée,
où, si X est un atome de carbone, chacun de R² à R⁷ est un groupe alkyle à chaîne droite ayant 1 à 4 atomes de carbone; chacun de R⁸ et R⁹ est un atome d'hydrogène; et R¹ est un groupe alkyle à chaîne droite ou à chaîne ramifiée ayant 1 à 10 atomes de carbone ou un groupe alcoxy à chaîne droite ou à chaîne ramifiée ayant 1 à 10 atomes de carbone ; et le composant anionique est (CF₃SO₂) ₂N⁻, PF₆⁻ ou BF₄⁻; ou
où, si X est un atome d'oxygène, chacun de R¹ à R⁷ est un groupe alkyle à chaîne droite ou à chaîne ramifiée ayant 1 à 10 atomes de carbone, et peuvent être identiques ou différents les uns des autres ; et le composant anionique représente un ou plusieurs type(s) choisi(s) dans le groupe constitué de [R^{f}SO₃]⁻, [(RfSO₂)₂N]⁻, CF₃SO₃⁻, CF₃COO⁻, PF₆⁻, BF₄⁻, [N(CN)₂]⁻, [AlCl₄]⁻, SO₄²⁻, HSO₄⁻, NO₃⁻, F⁻, Br⁻ et I⁻ ; où R^{f} est un groupe de substitution contenant du fluor.

2. Liquide ionique selon la revendication 1, où dans la formule générale (1), X est un atome d'oxygène, chacun de R² à R⁷ est un groupe alkyle à chaîne droite ayant 1 à 4 atomes de carbone ; R¹ est un groupe alkyle à chaîne droite ou à chaîne ramifiée ayant 1 à 10 atomes de carbone ; et le composant anionique est (CF₃SO₂) ₂N-, PF₆⁻ ou BF₄⁻.

3. Liquide ionique selon la revendication 1, dans lequel dans la formule générale (1), X est un atome de carbone, chacun de R² à R⁷ est un groupe alkyle à chaîne droite ayant 1 à 4 atomes de carbone ; chacun de R⁸ et R⁹ est un atome d'hydrogène ; et R¹ est un groupe alkyle à chaîne droite ou à chaîne ramifiée ayant 1 à 10 atomes de carbone ou un groupe alcoxy à chaîne droite ou à chaîne ramifiée ayant 1 à 10 atomes de carbone ; et le composant anionique est (CF₃SO₂)₂N⁻ou PF₆⁻.

4. Dispositif de stockage électrique comprenant le liquide ionique selon l'une des revendications 1 à 3 en tant que solution électrolytique.

5. Batterie au lithium rechargeable comprenant le liquide ionique selon l'une des revendications 1 à 3.

6. Condensateur électrique à double couche comprenant le liquide ionique selon l'une des revendications 1 à 3.

7. Cellule solaire sensibilisée par colorant comprenant le liquide ionique selon l'une des revendications 1 à 3.

8. Pile à combustible comprenant le liquide ionique selon l'une des revendications 1 à 3.

9. Solvant de réaction comprenant le liquide ionique selon l'une des revendications 1 à 3.

10. Procédé destiné à produire un liquide ionique comprenant l'alkylation d'une substance organique représentée par la formule générale suivante (2),
le liquide ionique contenant une substance organique représentée par la formule générale (1) en tant que composant cationique, 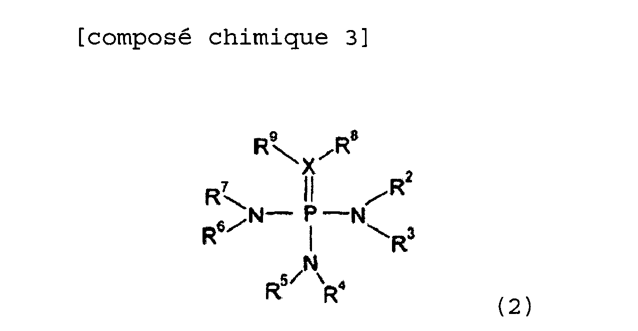 où des groupes de substitution de R² à R⁹ peuvent être indépendamment identiques ou différents les uns des autres; X représente un atome de carbone ou un atome d'oxygène; et R⁸ et R⁹ existent seulement lorsque X est un atome de carbone,
où, si X est un atome de carbone, chacun de R² à R⁷ est un groupe alkyle à chaîne droite ayant 1 à 4 atomes de carbone; chacun de R⁸ et R⁹ est un atome d'hydrogène; et R¹ est un groupe alkyle à chaîne droite ou à chaîne ramifiée ayant 1 à 10 atomes de carbone ou un groupe alcoxy à chaîne droite ou à chaîne ramifiée ayant 1 à 10 atomes de carbone; ou
où, si X est un atome d'oxygène, chacun de R² à R⁷ est un groupe alkyle à chaîne droite ou à chaîne ramifiée ayant 1 à 10 atomes de carbone, et peuvent être identiques ou différents les uns des autres.

11. Procédé destiné à produire un liquide ionique selon la revendication 10, comprenant un échange d'anions avec un sel résultant après l'alkylation.
